# EUROPEAN PATENT SPECIFICATION

(11) **EP 3 579 240 B1**
(45) Date of publication and mention of the grant of the patent: **24.07.2024**
(21) Application number: 18176468.9
(22) Date of filing: 07.06.2018
(51) Int. Cl.: G16H 10/60

(54) **METHOD AND SYSTEM FOR PROVIDING SECOND MEDICAL DATA RECORD**
VERFAHREN UND SYSTEM ZUR BEREITSTELLUNG EINER AUFZEICHNUNG VON MEDIZINISCHEN DATEN
PROCÉDÉ ET SYSTÈME DE FOURNITURE D'UN SECOND DOSSIER DE DONNÉES MÉDICALES

(43) Date of publication of application: 11.12.2019
(73) Proprietor: Siemens Healthineers AG, 91301 Forchheim (DE)
(72) Inventor: Athmanathan, Balakrishnan, 560061 Bangalore (IN); Rangapura Shettappa, Chandrashekara, 91058 Erlangen (DE); Thomas, Arun, 691507 Kollam, Kerala (IN)
(74) Representative: Bals & Vogel Patentanwälte PartGmbB

(56) References cited:
- US-A1- 2014 067 426
- US-A1- 2014 180 707
- US-A1- 2017 132 368

## Description

The invention relates to a method of providing a second medical data record. The method and system disclosed therein comprises the steps of receiving a first medical data record by a first interface, wherein the first medical data record is related to a patient; and determining a data record identifier from the first medical data record by a processing unit.

A medical data record is a documentation of a patient's health information, the medical history, observations recorded by a physician, drugs and therapies administered to the patient, etc. The medical data record also includes a physician's findings and analysis of several medical studies conducted on the patient. The medical studies may be, for example, diagnostic tests such as analysis of body fluids, medical imaging, etc. As the medical data record is usually a summarized documentation of a patient's health, it may not include all the details of the medical studies conducted on the patient. For example, the medical data record may include only one medical image, i.e. one view of the imaged body part. If the patient visits the physician again, or has to obtain a second opinion from another physician, the physician may not be able to provide an accurate analysis of the patient's condition based on a single medical image. The physician may need additional medical data to efficiently diagnose the patient's condition. If the physician wants to analyze other views of the image in the medical report it may be cumbersome to obtain the original image file, load the image and identify the region of interest and identify the interested view of the image.

US 2017/132368 A1 discloses a healthcare content management system where a request to process one or more images acquired by one or more devices is received.

US 2014/180707 A1 discloses a smartphone, tablet or in store kiosk application which allows a user to input information related to a pharmaceutical product and, in response, displays a list of interactions related to the pharmaceutical product and at least one other product.

US 2014/067426 A1 discloses a method and system that allows mobile or embedded devices to be dynamically paired with medical applications.

In the light of the above, there exists a need to provide a method and system for providing a second medical record that is accurately identifies and extracts the related second medical record associated with the first medical record.

The object of the invention is therefore to provide a method and a system for providing a second medical record which is accurate and convenient.

This object is solved by a method of providing a second medical record according to claim 1, a local transmission unit according to claim 10, a computer program product according to claim 11, and a computer readable medium according to claim 12.

In the following, the solution according to the invention is described with respect to the claimed local transmission unit as well as with respect to the claimed method. Features, advantages or alternative embodiments herein can be assigned to the other claimed objects and vice versa. In other words, claims for the local transmission unit can be improved with features described or claimed in the context of the method. In this case, the functional features of the method are embodied by objective units of the local transmission unit.

Based on the aforementioned method, the invention achieves the object by identifying the second medical data record within a repository based on the data record identifier by the processing unit, wherein second medical data record comprises the data record identifier in the repository and providing the second medical data record. An advantage of the invention is that the second medical data record is provided to the physician conveniently. Therefore, the analysis of the medical data record is more efficient. The physician can obtain and refer to additional medical information from the second medical data record, based on which a correct medical diagnosis may be made. Therefore, incorrect medical advice based on incomplete medical information is avoided.

The present invention describes a method of providing a second medical data record. According to an embodiment, the method comprises receiving a first medical data record by a first interface. The first medical data record is related to a patient. A medical data record is a documentation of a patient's medical history and care. Such documentation may be recorded over a period of time and may contain, for example, one or more medical images, diagnosis information, and therapy information. In an embodiment, the first medical data record includes an image of a physical medical data record, i.e., a medical data record in physical medium. The image of the physical medical data record may be, for example, a digital image. The first interface may be, for example, a network interface between a client device and a server. The method further comprises determining a data record identifier from the first medical data record by a processing unit. A data record identifier is a unique tag designated to every medical data record using which one medical data record is differentiated from another. Such a tag may be numerical, alphabetical, or a combination of both. Alternatively, the tag may be used in combination with the patient's name to identify the second medical data record. The method further comprises a step of identifying the second medical data record within a repository based on the data record identifier by the processing unit. The second medical data record may be, for example, but not limited to, one or more medical images of the patient that may not be present in the first medical data record. Such one or more medical images may contain, for example, one or more views of an imaged part of the patient's body. Additionally, the second medical data record may also contain annotations of the one or more views of the medical image. The second medical data record also contains the data record identifier, based on which the second medical data record is identified from the repository. The repository may be, for example, a medical database that contains information specific to a plurality of patients. Advantageously, the data record identifier enables accurate identification of any second medical data record that may be available in the repository. The advantage of identifying the second medical record is that a physician evaluating the medical data record of the patient is supplemented with additional medical information. Such additional medical information may enable the physician to accurately and holistically diagnose the patient's medical condition and provide correct medical advice and therapy. The method further comprises providing by the first interface the second medical data record. The advantage of the invention is that accurate diagnosis of the medical condition of the patient is enabled using the second medical data record. Additionally, provision of the second medical data record enables faster processing of the medical record by the by the physician. As the physician is supplemented with complete medical information, faster analysis of such medical information is possible. Furthermore, the patient may not carry along his/her medical data every time a consultation with a physician is planned.

According to an embodiment of the invention, in receiving the first medical record, the method includes capturing a digital image of the physical medical data record by a client device. In an embodiment, the physical medical data record may be a physical print medium, for example, the physical medical data record may be paper based. A digital image of such physical medical data record is captured by the client device. The client device may include a camera which may be used to capture the digital image of the physical medical record. Advantageously, a digital image of the physical medical record enables digitization of the print medium based physical medical data record. In an embodiment, the digital image of the physical medical data record may be stored in the medical database.

According to an embodiment of the invention, the first medical data record is processed by the processing unit. A portion of the first medical data record containing the data record identifier is identified. The data record identifier may be located in a specific portion of the first medical data record. The portion of the first medical data record containing the data record identifier may be identified, for example, based on a standard text associated with the data record identifier. For example, the data record identifier may be preceded by a text phrase "medical record number". The first medical data record may be scanned to identify the location of such standard text, based on which the portion of the first medical data record containing the data record identifier may be identified accurately. The data record identifier may be extracted by the processing unit from the identified portion by the processing unit. Advantageously, extraction of the data record identifier enables determination of the corresponding second medical data record from the repository.

According to another embodiment of the invention, the data record identifier is identified in the portion of the first medical data record by text information extraction of the image of the first medical data record. Text information extraction is a process of identifying text information from unstructured, semi-structured or structured text. Text information extraction may involve entity recognition and relation extraction. Advantageously, text information extraction enables identification of text in the first medical data record efficiently. In the entity recognition, the standard text associated with the data record identifier may be determined, following which the data record identifier may be identified using relation extraction.

According to an embodiment of the invention, the first medical data record may be processed to detect the presence of text information. The text in the digital image is localized to accurately determine the portion of the digital image which contains the data record identifier. The text in the image is extracted by the processing unit and recognized using optical character recognition (OCR). OCR is a method of conversion of printed or handwritten text to an electronic form or machine-encoded text. Such conversion may be made from a digital image of the printed or handwritten text. The steps involved in OCR are well-known in the art. The advantage of the method is that the data record identifier is accurately localized in the first medical record, thereby enabling precise extraction of the data record identifier. Conversion of printed text to machine encoded text is enabled using which the data record identifier is easily extracted from the first medical data record. Therefore, the step of manual entry of the data record identifier in a processing system to identify the second medical data record is eliminated. Advantageously, the determination of the second medical data record is enabled using a physical print medium.

According to yet another embodiment of the invention, the data record identifier may be encoded in a one-dimensional or a two-dimensional code in the first medical record. Such one-dimensional or two-dimensional code may be optical and machine-readable. For example, the one-dimensional code may be, but not limited to, a barcode. The two dimensional code may be, for example, but not limited to, QR-code, Aztec code, and Data Matrix code. The data record identifier may be extracted from the one-dimensional or two-dimensional code by the processing unit.

According to a preferred embodiment of the invention, the method further comprises augmenting data from the second medical data record on the first medical data record by the processing unit. In an embodiment, the medical data present in the second medical data record may be extracted and augmented on to the first medical data record. Such augmentation may include, for example, annotating a medical image in the first medical data record with additional information from the second medical data record or replacing the medical image in the first medical data record with a DICOM image from the second medical data record. Alternatively, metadata associated with medical data in the second medical data record may be added as annotations in the first medical data record. In an embodiment, the extracted medical data from the second medical data record may be annotated on the first medical data record as a text note. Such text note may be placed adjacent to the medical data in the first medical data record. Advantageously, the medical information present in the first medical data record is updated using the medical data from the second medical data record. Therefore, all the medical information required for the physician to make an informed diagnosis is available on a single medical data record.

According to an embodiment not part of the invention, in augmenting the first medical data record, a first keyword is identified in the first medical data record, by the processing unit. The keyword may be, for example, specific to the medical information recorded in the first medical data record. In an embodiment, the first keyword may be identified from the first medical data record using optical character recognition. The identified first keyword is correlated with a corresponding second keyword in the second medical data record by the processing unit. The second medical data record may be processed to identify the second keyword corresponding to the first keyword identified from the first medical data record. The corresponding second keyword may be identified based on similarity. Alternatively, the keywords may also be identified based on their relation with each other. Correlating keywords from the first medical data record and the second medical data record advantageously enables accurate determination of data to be augmented on the first medical data record. The method further includes extracting a data corresponding to the correlated second keyword in the second medical data record by the processing unit. The correlated second keyword may be associated with a medical information in the first and second medical data record. Therefore, the correlated keywords enable determination of medical information which is to be augmented on the first medical data record. The data may be extracted in the form of an image or text for augmentation. In an embodiment, the extracted data from the second medical data record may be metadata associated with the medical data associated with the second keyword. Such metadata may be extracted using techniques well-known the state of the art. The method further comprises annotating extracted data from the second medical data record on the first medical data record. The annotation on the first medical data record may be performed in the same location as that of the first keyword. Alternatively, the annotation may be performed adjacent to the location of the first keyword in the first medical data record. The advantage of the method is that the first medical data record is supplemented with additional medical information. This enables the physician to accurately diagnose the medical condition of the patient. Advantageously, the method enables faster diagnosis by reducing the wait time to obtain all the medical information required to make a correct decision.

According to the invention, in augmenting the first medical data record, the method comprises identifying a first attribute by the processing unit from the first medical data record. The first attribute may be a patient identification number. Alternatively, the first attribute may also be type of imaging modality. A corresponding second medical image is determined from the second medical data record by the processing unit, based on the identified first attribute. In an embodiment, the second medical image is a DICOM image. The corresponding DICOM image may be determined by adjusting the scaling and view of the DICOM image, such that a match with the first medical image is determined. In an embodiment, such second medical image may comprise alternate views of the object imaged in the first medical image. The second medical image is extracted from the second medical data record by the processing unit and the first medical data record is augmented with the second medical image. In an embodiment, the second medical image may be superimposed on the first medical image in the first medical data record. Alternatively, the second medical image may be placed adjacent to the first medical image. The advantage of the method is that alternate views of the imaged object are provided to the physician. This enables the physician to make an accurate diagnosis of the patient's condition. Another advantage of the method is that augmentation of the second medical image with the first medical data record enables the doctor to have all the necessary information in one medical report. Therefore, the need to search the repository each time for medical data is eliminated. Yet another advantage of the method is that the time taken for efficient diagnosis is reduced, thereby enabling faster treatment.

According to an embodiment not part of the invention, the correlating keyword in the first medical data record and the second medical data record is determined by the processing unit based on historical data of correlating keywords. The historical data may be derived, for example, based on a user action. In an embodiment, a newly correlated first keyword and second keyword may be stored in a medical database each time a request to provide a second medical data record is processed by the processing unit. The correlation between the first and the second keyword may be determined manually for the first time, i.e. based on a user action. Such user action may define a relationship between the first keyword and the second keyword. Alternatively, the correlation between the first and the second keyword may also be rule based. For example, the correlation between the first and the second keyword may be determined based on the similarity between the first and the second keywords. In an embodiment, the processing unit may be configured to learn the relation between a first keyword and a second keyword, thereby enabling faster correlation of the second keyword from the second medical data record based on the first keyword. In another embodiment, the processing unit is further configured to generate new rules and/or to update the existing rules based on the user actions so as to correlate the first keyword with the second keyword. Advantageously, determination of correlation using historical data ensures accurate identification of corresponding second keyword in the second medical data record.

According to another embodiment of the invention, the data record identifier may be, for example, but not limited to, patient name, medical data record number, and/or patient health information.

According to yet another embodiment of the invention, the method further comprises receiving a request to access the second medical data record by a server. A user or a physician may request an access to the second medical data record in the medical database. On receiving such a request, the server is configured to authenticate the request to identify if the request has been received from an authorized source. On successful authentication, the server is further configured to provide the authorized source access to the second medical data record. In an embodiment, such a request may be received from a user or a physician who may be registered to a portal associated with the medical database that enables access to the medical data records. Advantageously, this step enables maintenance of security of the medical data record.

The invention also relates to a local transmission unit configured for providing a second medical data record. The local transmission unit comprises a first interface configured to receive a first medical data record from a source. In an embodiment, the first medical data record may be an image of a physical medical data record, i.e. a medical data record in physical print medium. The image of the physical data record may be, for example, a digital image. The source may be a client device, for example, a handheld device. The first interface may be, for example, a network interface between the client device and a server. The local transmission unit further comprises a processing unit configured to determine a data record identifier from the first medical data record. A data record identifier is a unique tag designated to every medical data record using which one medical data record is differentiated from another. Such a tag may be numerical, alphabetical, or a combination of both. Alternatively, the tag may be used in combination with the patient's name to identify the second medical data record. The processing unit may further be configured to identify the second medical data record within a repository based on the data record identifier by the processing unit. The second medical data record may be, for example, but not limited to, one or more medical images of the patient that may not be present in the first medical data record. Such one or more medical images may contain, for example, one or more views of an imaged part of the patient's body. Additionally, the second medical data record may also contain annotations of the one or more views of the medical image. The second medical data record also contains the data record identifier, based on which the second medical data record is identified from the repository. The repository may be, for example, a medical database that contains information specific to a plurality of patients. Advantageously, the data record identifier enables accurate identification of any second medical data record that may be available in the repository. The advantage of identifying the second medical record is that a physician evaluating the medical data record of the patient is supplemented with additional medical information. Such additional medical information may enable the physician to accurately and holistically diagnose the patient's medical condition and provide correct medical advice and therapy. The processing unit is further configured to provide by the first interface the second medical data record. The advantage of the invention is that accurate diagnosis of the medical condition of the patient is enabled using the second medical data record. Additionally, provision of the second medical data record enables faster processing of the medical record by the by the physician. As the physician is supplemented with complete medical information, faster analysis of such medical information is possible. Furthermore, the patient may not carry along his/her medical data every time a consultation with a physician is planned.

According to an embodiment of the invention, in receiving the first medical record, the processing unit is configured to capture a digital image of the physical medical data record by a client device. In an embodiment, the physical medical data record may be a physical print medium, for example, the first medical data record may be paper based. A digital image of such physical medical record is captured by the client device. The client device may include a camera which may be used to capture the digital image of the physical medical data record. Advantageously, a digital image of the physical medical record enables digitization of the print medium based physical medical data record. In an embodiment, the digital image of the physical medical data record may be stored in the medical database.

According to an embodiment of the invention, the first medical data record is processed by the processing unit. A portion of the first medical data record containing the data record identifier is identified. The data record identifier may be located in a specific portion of the first medical data record. The portion of the first medical data record containing the data record identifier may be identified, for example, based on a standard text associated with the data record identifier. For example, the data record identifier may be preceded by a text phrase "medical record number". The first medical data record may be analyzed to identify the location of such standard text, based on which the portion of the first medical data record containing the data record identifier may be identified accurately. The data record identifier may be extracted by the processing unit from the identified portion by the processing unit. Advantageously, extraction of the data record identifier enables determination of the corresponding second medical data record from the repository.

According to another embodiment of the invention, the processing unit is configured to identify the data record identifier in the portion of the first medical data record by text information extraction of the image of the first medical data record. Text information extraction is a process of identifying text information from unstructured, semi-structured or structured text. Text information extraction may involve entity recognition and relation extraction. Advantageously, text information extraction enables identification of text in the first medical data record efficiently. In the entity recognition, the standard text associated with the data record identifier may be determined, following which the data record identifier may be identified using relation extraction.

According to an embodiment of the invention, the processing unit may be configured to process the first medical data record to detect the presence of text information. The text in the digital image is localized to accurately determine the portion of the digital image which contains the data record identifier. The text in the image is extracted by the processing unit and recognized using optical character recognition (OCR). OCR is a method of conversion of printed or handwritten text to an electronic form or machine-encoded text. Such conversion may be made from a digital image of the printed or handwritten text. The steps involved in OCR are well-known in the art. The advantage of the method is that the data record identifier is accurately localized in the first medical record, thereby enabling precise extraction of the data record identifier. Conversion of printed text to machine encoded text is enabled using which the data record identifier is easily extracted from the first medical data record. Therefore, the step of manual entry of the data record identifier in a processing system to identify the second medical data record is eliminated. Advantageously, the determination of the second medical data record is enabled using a physical print medium.

According to yet another embodiment of the invention, the data record identifier may be encoded in a one-dimensional or a two-dimensional code in the first medical record. Such one-dimensional or two-dimensional code may be optical and machine-readable. For example, the one-dimensional code may be, but not limited to, a barcode. The two dimensional code may be, for example, but not limited to, QR-code, Aztec code, and Data Matrix code. The data record identifier may be extracted from the one-dimensional or two-dimensional code by the processing unit.

According the invention, the processing unit is further configured to augment data from the second medical data record on the first medical data record. In an embodiment, the medical data present in the second medical data record may be extracted and augmented on to the first medical data record. Such augmentation may include, for example, annotating a medical image in the first medical data record with additional information from the second medical data record or replacing the medical image in the first medical data record with a DICOM image from the second medical data record. Alternatively, metadata associated with medical data in the second medical data record may be added as annotations in the first medical data record. In an embodiment, the extracted medical data from the second medical data record may be annotated on the first medical data record as a text note. Such text note may be placed adjacent to the medical data in the first medical data record. Advantageously, the medical information present in the first medical data record is updated using the medical data from the second medical data record. Therefore, all the medical information required for the physician to make an informed diagnosis is available on a single medical data record.

According to an embodiment not part of the invention, in augmenting the first medical data record, the processing unit is configured to identify a first keyword in the first medical data record. The keyword may be, for example, specific to the medical information recorded in the first medical data record. In an embodiment, the first keyword may be identified from the first medical data record using optical character recognition. The identified first keyword is correlated with a corresponding second keyword in the second medical data record by the processing unit. The second medical data record may be processed to identify the second keyword corresponding to the first keyword identified from the first medical data record. The corresponding second keyword may be identified based on similarity. Alternatively, the keywords may also be identified based on their relation with each other. Correlating keywords from the first medical data record and the second medical data record advantageously enables accurate determination of data to be augmented on the first medical data record. The processing unit is further configured to extract a data corresponding to the correlated second keyword in the second medical data record. The correlated second keyword may be associated with medical information in the first and second medical data record. Therefore, the correlated keywords enable determination of medical information which is to be augmented on the first medical data record. The data may be extracted in the form of an image or text for augmentation. In an embodiment, the extracted data from the second medical data record may be metadata associated with the medical data associated with the second keyword. Such metadata may be extracted using techniques well-known the state of the art. The processing unit is further configured to annotate extracted data from the second medical data record on the first medical data record. The annotation on the first medical data record may be performed in the same location as that of the first keyword. Alternatively, the annotation may be performed adjacent to the location of the first keyword in the first medical data record. The advantage of the invention is that the first medical data record is supplemented with additional medical information. This enables the physician to accurately diagnose the medical condition of the patient. Advantageously, the invention enables faster diagnosis by reducing the wait time to obtain all the medical information required to make a correct decision.

According to the invention, in augmenting the first medical data record, the processing unit is configured to identify a first attribute by the processing unit from the first medical data record. The first attribute may be a patient identification number. Alternatively, the first attribute may also be type of imaging modality. The processing unit is further configured to determine a corresponding second medical image from the second medical data record, based on the identified first attribute. In an embodiment, the second medical image is a DICOM image. The corresponding DICOM image may be determined by adjusting the scaling and view of the DICOM image, such that a match with the first medical image is determined. In an embodiment, such second medical image may comprise alternate views of the object imaged in the first medical image. The second medical image is extracted from the second medical data record by the processing unit and the first medical data record is augmented with the second medical image. In an embodiment, the second medical image may be superimposed on the first medical image in the first medical data record. Alternatively, the second medical image may be placed adjacent to the first medical image. The advantage of the invention is that alternate views of the imaged object are provided to the physician. This enables the physician to make an accurate diagnosis of the patient's condition. Another advantage of the invention is that augmentation of the second medical image with the first medical data record enables the doctor to have all the necessary information in one medical report. Therefore, the need to search the repository each time for medical data is eliminated. Yet another advantage of the invention is that the time taken for efficient diagnosis is reduced, thereby enabling faster treatment.

According to an embodiment not part of the invention, the processing unit is configured to determine correlating keyword in the first medical data record and the second medical data record based on historical data of correlating keywords. The historical data may be derived, for example, based on a user action. In an embodiment, a newly correlated first keyword and second keyword may be stored in a medical database each time a request to provide a second medical data record is processed by the processing unit. The correlation between the first and the second keyword may be determined manually for the first time, i.e. based on a user action. Such user action may define a relationship between the first keyword and the second keyword. Alternatively, the correlation between the first and the second keyword may also be rule based. For example, the correlation between the first and the second keyword may be determined based on the similarity between the first and the second keywords. In an embodiment, the processing unit may be configured to learn the relation between a first keyword and a second keyword, thereby enabling faster correlation of the second keyword from the second medical data record based on the first keyword. In another embodiment, the processing unit is further configured to generate new rules and/or to update the existing rules based on the user actions so as to correlate the first keyword with the second keyword. Advantageously, determination of correlation using historical data ensures accurate identification of corresponding second keyword in the second medical data record.

According to another embodiment of the invention, the data record identifier may be, for example, but not limited to, patient name, medical data record number, and/or patient health information.

According to yet another embodiment of the invention, the processing unit is further configured to receive a request to access the second medical data record by a server. A user or a physician may request an access to the second medical data record in the medical database. On receiving such a request, the server is configured to authenticate the request to identify if the request has been received from an authorized source. On successful authentication, the server is further configured to provide the authorized source access to the second medical data record. In an embodiment, such a request may be received from a user or a physician who may be registered to a portal associated with the medical database that enables access to the medical data records. Advantageously, this step enables maintenance of security of the medical data record.

The invention relates in one aspect to a computer program product comprising a computer program, the computer program being loadable into a storage unit of a local transmission unit, including program code sections to make the local transmission unit execute the method according to an aspect of the invention when the computer program is executed in the local transmission unit.

The invention relates in one aspect to a computer-readable medium, on which program code sections of a computer program are saved, the program code sections being loadable into and/or executable in a local transmission unit to make the local transmission unit execute the method according to an aspect of the invention when the program code sections are executed in the local transmission unit.

The realization of the invention by a computer program product and/or a computer-readable medium has the advantage that already existing allocation systems can be easily adopted by software updates in order to work as proposed by the invention.

The computer program product can be, for example, a computer program or comprise another element apart from the computer program. This other element can be hardware, for example a memory device, on which the computer program is stored, a hardware key for using the computer program and the like, and/or software, for example a documentation or a software key for using the computer program.

The present invention is further described hereinafter with reference to illustrated embodiments shown in the accompanying drawings, in which:
- FIG 1: illustrates a block diagram of a client-server architecture which provides geometric modeling of components representing different parts of a real world object, according to an embodiment.
- FIG 2: illustrates a block diagram of a local transmission unit in which an embodiment of a method for providing a second medical data record can be implemented.
- FIG 3: illustrates a flowchart of an embodiment of a method of providing a second medical data record.
- FIG 4: illustrates a flowchart of an embodiment of a method of determining a data record identifier in the first medical data record.
- FIG 5: illustrates a flowchart of an embodiment of a method of extracting text from an image.
- FIG 6: illustrates a flowchart of another embodiment of a method of augmenting data from second medical data record on the first medical data record.
- FIG 7: illustrates a flowchart of another embodiment of a method of augmenting data from second medical data record on the first medical data record.
- FIG 8: illustrates a flowchart of an embodiment of a method of authenticating a request to access the second medical data record.

Hereinafter, embodiments for carrying out the present invention are described in detail. The various embodiments are described with reference to the drawings, wherein like reference numerals are used to refer to like elements throughout. In the following description, for purpose of explanation, numerous specific details are set forth in order to provide a thorough understanding of one or more embodiments. It may be evident that such embodiments may be practiced without these specific details.

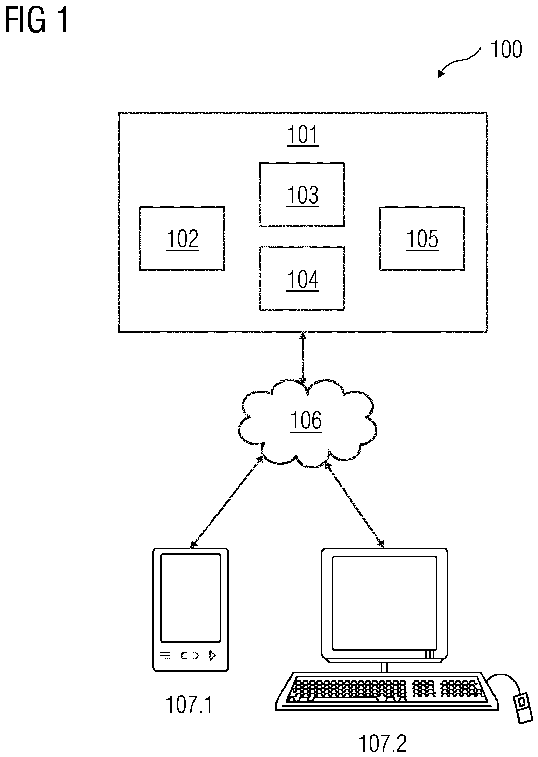

FIG 1 provides an illustration of a block diagram of a client-server architecture that is a geometric modelling of components representing different parts of real-world objects, according to an embodiment. The client-server architecture 100 includes a server 101 and a plurality of client devices 107.1-107.2. Each of the client devices 107.1-107.2 are connected to the server 101 via a network 106, for example, local area network (LAN), wide area network (WAN), WiFi, etc. In one embodiment, the server 101 is deployed in a cloud computing environment. As used herein, "cloud computing environment" refers to a processing environment comprising configurable computing physical and logical resources, for example, networks, servers, storage, applications, services, etc., and data distributed over the network 106, for example, the in-ternet. The cloud computing environment provides on-demand network access to a shared pool of the configurable computing physical and logical resources. The server 101 may include a medical database 102 that comprises medical data records of a plurality of patients. The medical database may contain medical data obtained from, but not limited to, one or more medical equipment, for example scanners, hospitals, medical professionals, and other providers that may be involved in patient care. The medical database may obtain patient medical information from, for example, PACS (acronym for "Picture Archiving and Communication System"), HIS (acronym for "Hospital Information System"), LIS (acronym for "Laboratory Information System") and RIS (acronym for "Radiology Information System"). The server 101 may include a data record module 103 that provides a second medical data record and augments data from the second medical data record on a first medical data record. The server 101 may also include a security module 104 that maintains the security of the medical data record. Additionally, the server 101 may include a network interface 107 for communicating with the client devices 107.1-107.2 via the network 106.

The client devices 107.1-107.2 include a user device 107.1, used by a user. In an embodiment, the user device 107.1 may be used by the user, for example a patient, to capture a digital image of a physical medical data record, i.e. a medical data record in physical print medium. The digital image of the physical medical data record can be accessed by the user via a graphical user interface of an end user web application on the user device. In an embodiment, the digital image of the physical medical data record may be sent to the server 101 via the end user web application. In another embodiment, a request may be sent to the server 101 to access the second medical data record based on the digital image of the physical medical data record from another user, for example a physician, via the network 106.

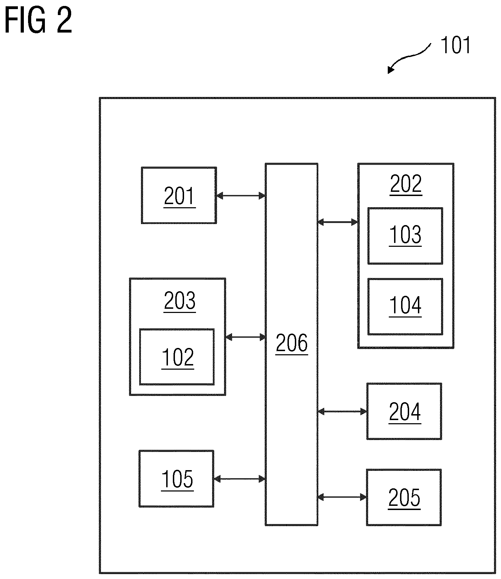

FIG 2 is a block diagram of a local transmission unit 101 in which an embodiment can be implemented, for example, as a system to provide a second medical data record, configured to perform the processes as described therein. It is appreciated that the server 101 is an exemplary implementation of the local transmission unit in FIG 2. In FIG 2, the local transmission unit 101 comprises a processing unit 201, a memory 202, a storage unit 203, an input unit 204, an output unit 205 a network interface 105 and a standard interface or bus 206. The local transmission unit 101 can be a (personal) computer, a workstation, a virtual machine running on host hardware, a microcontroller, or an integrated circuit. As an alternative, the local transmission unit 101 can be a real or a virtual group of computers (the technical term for a real group of computers is "cluster", the technical term for a virtual group of computers is "cloud").

The processing unit 201, as used herein, means any type of computational circuit, such as, but not limited to, a microprocessor, microcontroller, complex instruction set computing microprocessor, reduced instruction set computing microprocessor, very long instruction word microprocessor, explicitly parallel instruction computing microprocessor, graphics processor, digital signal processor, or any other type of processing circuit. The processing unit 201 may also include embedded controllers, such as generic or programmable logic devices or arrays, application specific integrated circuits, single-chip computers, and the like. In general, a processing unit 201 can comprise hardware elements and software elements. The processing unit 201 can be configured for multi-threading, i.e. the processing unit 201 can host different calculation processes at the same time, executing the either in parallel or switching between active and passive calculation processes.

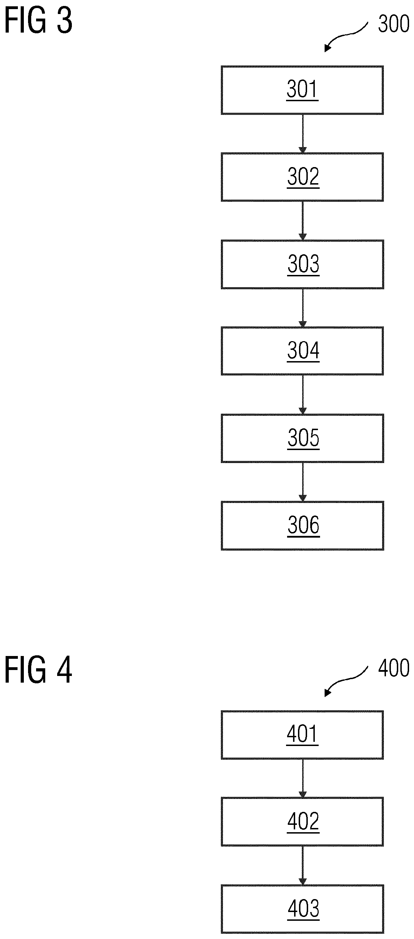

The memory 202 may be volatile memory and non-volatile memory. The memory 202 may be coupled for communication with the processing unit 201. The processing unit 201 may execute instructions and/or code stored in the memory 202. A variety of computer-readable storage media may be stored in and accessed from the memory 202. The memory 202 may include any suitable elements for storing data and machine-readable instructions, such as read only memory, random access memory, erasable programmable read only memory, electrically erasable programmable read only memory, a hard drive, a removable media drive for handling compact disks, digital video disks, diskettes, magnetic tape cartridges, memory cards, and the like. In the present embodiment, the memory 202 includes a data record module 103, and a security module 104 stored in the form of machine-readable instructions on any of the above-mentioned storage media and may be in communication to and executed by processing unit 201. When executed by the processing unit 201, the data record module 103 causes the processing unit 201 to analyze the first medical data record and provide a second medical data record from the medical database 102. The data record module may further cause the processing unit to augment data from the second medical data record on the first medical data record. When executed by the processing unit 201, the security module 104 causes the processing unit 201 to authenticate any request received to access to the second medical data record. Method steps executed by the processing unit 201 to achieve the abovementioned functionality are elaborated upon in detail in Figure 3, 4, 5, 6, 7 and 8.

The storage unit 203 may be a non-transitory storage medium which stores a medical database 102. The medical database 102 is a repository of medical information related to one or more patients that is maintained by a healthcare service provider. The input unit 204 may include input means such as keypad, touch-sensitive display, camera (such as a camera receiving gesture-based inputs), etc. capable of receiving input signal. The bus 207 acts as interconnect between the processing unit 201, the memory 202, the storage unit 203, the communication interface 107 the input unit 204 and the output unit 205.

Those of ordinary skilled in the art will appreciate that the hardware depicted in FIG 2 may vary for particular implementations. For example, other peripheral devices such as an optical disk drive and the like, Local Area Network (LAN)/ Wide Area Network (WAN)/ Wireless (e.g., Wi-Fi) adapter, graphics adapter, disk controller, input/output (I/O) adapter also may be used in addition or in place of the hardware depicted. The depicted example is provided for the purpose of explanation only and is not meant to imply architectural limitations with respect to the present disclosure.

A local transmission unit in accordance with an embodiment of the present disclosure includes an operating system employing a graphical user interface. The operating system permits multiple display windows to be presented in the graphical user interface simultaneously with each display window providing an interface to a different application or to a different instance of the same application. A cursor in the graphical user interface may be manipulated by a user through the pointing device. The position of the cursor may be changed and/or an event such as clicking a mouse button, generated to actuate a desired response.

One of various commercial operating systems, such as a version of Microsoft Windows^{™}, a product of Microsoft Corporation located in Redmond, Washington may be employed if suitably modified. The operating system is modified or created in accordance with the present disclosure as described.

The present invention is not limited to a particular computer system platform, processing unit, operating system, or network. One or more aspects of the present invention may be distributed among one or more computer systems, for example, servers configured to provide one or more services to one or more client computers, or to perform a complete task in a distributed system. For example, one or more aspects of the present invention may be performed on a client-server system that comprises components distributed among one or more server systems that perform multiple functions according to various embodiments. These components comprise, for example, executable, intermediate, or interpreted code, which communicate over a network using a communication protocol. The present invention is not limited to be executable on any particular system or group of systems, and is not limited to any particular distributed architecture, network, or communication protocol.

Disclosed embodiments provide systems and methods for analysing a medical data record. In particular, the systems and methods may provide a second medical data record based on the first medical data record.

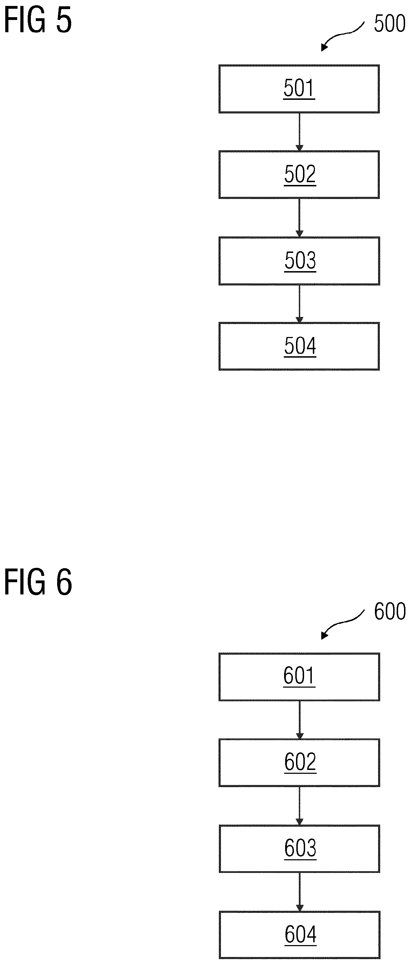

FIG 3 illustrates an embodiment of a flowchart of a method 300 of providing a second medical data record. At step 301 of the method 300, a digital image of a physical medical data record is captured. The physical medical data record may be a physical print medium. In an embodiment, the physical medical data record is paper based. The digital image of the physical medical data record is captured using a client device or user device 107.1. The client device may include a camera which may be used to capture the digital image of the physical medical record. In an embodiment, a first medical data record is the digital image of the physical medical record. The user device 107.1 may be a handheld device or a mobile device with an integrated camera. The user device 107.1 may comprise an imaging module associated with the camera and is capable of capturing an image of the physical medical data record. In an embodiment, the imaging module may also be configured to enhance the digital image of the physical medical data record so as to improve the image clarity. The digital image of the physical medical data record may be sent to the server 101 by the user through a web based application available on the user device 107.1, via a network 106. At step 302, the first medical data record is received by the processing unit 201 of the local transmission unit 101 via a network interface 105. At step 303, a data record identifier is determined from the first medical data record. A data record identifier is a unique tag designated to every medical data record using which one medical data record is differentiated from another. Such a tag is numerical, alphabetical, or alphanumerical. The data record identifier may be, for example, a patient identification number, patient's name, medical data record number and/or patient health information. The data record identifier enables identification of a corresponding second medical data record from the medical database 102. FIG 4 illustrates a flowchart of an embodiment of a method 400 of determining a data record identifier from the first medical data record. At step 401 of the method 400, the first medical data record is processed by the processing unit 201 to analyze the content of the image. At step 402, a portion of the first medical data record containing the data record identifier is identified. The data record identifier may be located in a specific portion of the first medical data record. In an embodiment, the data record identifier is identified in the digital image based on standard text associated with the data record identifier. The data record identifier may be preceded by a text phrase that enables identification of the data record identifier. In an embodiment, the data record identifier is preceded by a text phrase "medical record number". The first medical data record is analyzed to identify the preceding text and thereafter identify the data record identifier. At step 403, the data record identifier may be determined and extracted by the processing unit 201 from the first medical data record. The method steps of extracting text from the first medical data record are illustrated in detail in FIG 5. At step 501, the presence of text in the first medical data record is detected by the processing unit 201. In an embodiment, the digital image is analyzed using text information extraction method. Text information extraction is the process of identifying text information from unstructured, semi-structured or structured text. Text information extraction enables entity recognition and relation extraction from the digital image. Therefore, the data record module 103 is configured to recognize the entity, i.e. the standard text that precedes the data record identifier. Thereafter, the module 103 is configured to identify the data record identifier by relation extraction. At step 502, the text in the image is localized and at step 503, the text is extracted from the digital image. At step 504, the text is recognized using optical character recognition (OCR). OCR is a method of conversion of printed or handwritten text to an electronic form or machine encoded-text. Such conversion may be made from a digital image of the printed or handwritten text. The steps involved in OCR are well-known in the art. Alternatively, the data record identifier may be encoded in a one-dimensional or a two-dimensional code in the first medical record. Such one-dimensional or two-dimensional code may be optical and machine-readable. For example, the one-dimensional code may be, but not limited to, a barcode. The two dimensional code may be, for example, but not limited to, QR-code, Aztec code, and Data Matrix code. The data record identifier may be extracted from the one-dimensional or two-dimensional code by the processing unit 201.

At step 304 of the method 300, the second data record is identified from the medical database 102 based on the data record identifier in the first medical data record. The second medical data record contains an identical data record identifier as that identified in the first medical data record. The data record identifier enables accurate identification of the second medical data record so as to supplement medical data to the first medical data record. At step 305, the second medical data record is provided to the user by the processing unit 201. At step 306, the medical data from the second medical data record is augmented on the first medical data record.

FIG 6 illustrates an embodiment of a method 600 of augmenting data from the second medical data record on the first medical data record. At step 601, the first keyword in the first medical data record is identified by the data record module 103. In an embodiment, the first keyword is specific to a medical information associated with a patient. Such medical information is recorded in the first medical data record. The first keyword is identified using optical character recognition (OCR). At step 602, the identified keyword from the first medical data record is correlated with a corresponding second keyword in the second medical data record. The second medical data record is analyzed to identify a second keyword correlating with the first keyword. The correlation may be performed based on a defined rule. For example, the first keyword and the second keyword may be correlated based on similarity between the first and the second keywords. Alternatively, the correlation may also be performed based on the relation between the first keyword and the second keyword. In an embodiment, the relation between the first keyword and the second keyword is determined based on historical data of correlating keywords. The historical data may be derived, for example, based on a user action. In an embodiment, a newly correlated first keyword and second keyword may be stored in the medical database 102 each time a request to provide a second medical data record is processed by the processing unit 201. Therefore, the data record module is configured to store relational information associated with correlated keywords. Therefore, such relational information may be used to identify correlating keywords subsequently. In an embodiment, a new set of correlating keywords may be determined manually if historical data for such keywords is unavailable. In an embodiment, the processing unit 201 may be configured to learn the relation between a first keyword and a second keyword, thereby enabling faster correlation of the second keyword from the second medical data record based on the first keyword. In another embodiment, the processing unit 201 is further configured to generate new rules and/or to update the existing rules based on the user actions so as to correlate the first keyword with the second keyword. At step 603, data associated with the second keyword in the second medical data record is extracted. In an embodiment, the data associated with the second keyword is medical information associated with the patient. Such medical information corresponds to medical information in the first medical data record. The extracted data is in the form of text and at step 604 such extracted data is annotated on to the first medical data record. The annotation may be in the form of a text note on the first medical data record. Such text note may be placed adjacent to the medical data in the first medical data record.

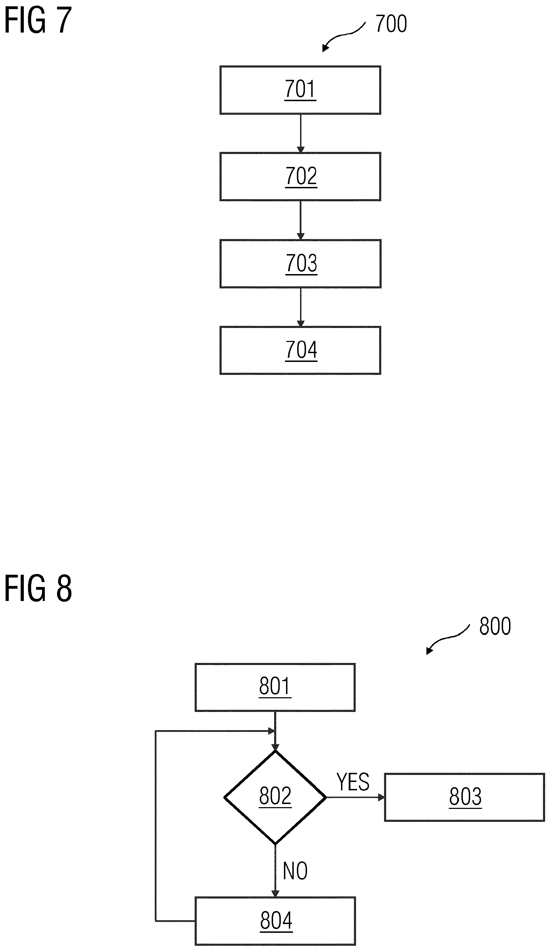

FIG 7 illustrates an embodiment of another method 700 of augmenting data from the second medical data record on the first medical data record. At step 701, a first attribute is identified from the first medical data record. In an embodiment, the first attribute is data associated with an imaged object in a first medical image in the first medical data record. Alternatively, the first attribute may also be type of imaging modality used or patient identification number. The first attribute associated with the imaged object is identified using OCR. At step 702, the data record module is configured to determine a second medical image in the second medical data record based on the identified first attribute. The second medical image is a Digital Imaging and Communications in Medicine (DICOM) image and comprises alternate views of the first medical image. In an embodiment, the corresponding second medical image is identified by adjusting the scaling of the DICOM image. At step 703, the second medical image is extracted from the second medical data record. The data record module is configured to extract one or more medical images of the imaged object. In an embodiment, the one or more medical images to be extracted may be identified by the physician who intends to analyze the first medical data record. Based on the input obtained from the physician, the data record module extracts the corresponding one or more DICOM images from the second medical data record. In an alternate embodiment, one or more views of the second medical image are set as default for extraction. The data record module 103 is configured to store the user input and learn the relation between the user input and the data extracted from the second medical data record so as to automatically extract relevant medical images from the second medical data record. At step 704, the second medical image is augmented on the first medical data record. In an embodiment, the second medical image is superimposed on the first medical image in the first medical data record. Alternatively, the second medical image is placed adjacent to the first medical image.

FIG 8 illustrates an embodiment of a method 800 of authenticating a request to access the second medical data record. At step 801, a request to access the second medical data record is received by the server 101. The user or the physician may request access to the second medical data record in the medical database 102. At step 802, the request is authenticated by the security module 104 to identify if the request has been received from an authorized source. In an embodiment, an authorized source is a user registered with an end user web application. The end user web application may prompt the user to input an access credential to obtain access to the second medical data record. The access credential may be, for example, a one-time password. The security module 104 is configured to generate a one-time password and send such one-time password to a registered mobile phone number of the user. On successful authentication, at step 803, the authorized source is provided access to the second medical data record. If the authentication is unsuccessful, at step 804, the access to the second medical data record is denied and the user is prompted to provide access credential again.

The foregoing examples have been provided merely for the purpose of explanation and are in no way to be construed as limiting of the present invention disclosed herein. While the invention has been described with reference to various embodiments, it is understood that the words, which have been used herein, are words of description and illustration, rather than words of limitation. Further, although the invention has been described herein with reference to particular means, materials, and embodiments, the invention is not intended to be limited to the particulars disclosed herein; rather, the invention extends to all functionally equivalent structures, methods and uses, such as are within the scope of the appended claims. Those skilled in the art, having the benefit of the teachings of this specification, may effect numerous modifications thereto and changes may be made without departing from the scope of the invention in its aspects.

## Claims

1. A method for providing a second medical data record, the method comprising:
Receiving (302) a first medical data record by a first interface (105), wherein the first medical data record is related to a patient;
determining (303) a data record identifier from the first medical data record by a processing unit (201), wherein the data record identifier is a unique numerical, alphabetical, or alphanumerical tag for differentiating one medical data record from another;
identifying (304) the second medical data record within a repository based on the data record identifier by the processing unit (201),
wherein second medical data record in the repository comprises the data record identifier; and
providing (305) the second medical data record by the first interface (105),
augmenting (306) data from the second medical data record on the first medical data record by the processing unit (201),
wherein in augmenting (306) data from the second medical data record, the method comprises:
identifying a first attribute in a first medical image associated with the first medical data record, wherein the first attribute may be a patient identification number or type of imaging modality;
determining a second medical image in the second medical data record corresponding to the first medical image in the first medical data record based on the first attribute;
extracting the second medical image from the second medical data record; and
augmenting the first medical data record with the second medical image.

2. The method according to claim 1, wherein the first medical data record comprises an image of a physical medical data record, wherein the image is a digital image.

3. The method according to claim 1, wherein in receiving the first medical data record, the method comprises capturing (301) a digital image of the physical medical data record by a client device (107).

4. The method according to claim 2, wherein in determining the data record identifier, the method comprises:
processing (401) the first medical data record by the processing unit (201);
identifying (402) a portion of the first medical data record containing the data record identifier from the image of the first medical data record by the processing unit (201); and
extracting (403) the data record identifier from the identified portion by the processing unit (201).

5. The method according to one of the preceding claims, wherein the data record identifier is identified in the portion of the first medical record by text information extraction of the image of the first medical data record.

6. The method of claim 5, wherein the text information extraction comprises:
detecting (501) the presence of a text in the first medical data record by the processing unit (201);
localizing (502) the text in the first medical data record by the processing unit (201);
extracting (503) the text in the first medical data record by the processing unit (201); and
recognizing (504) the text in the first medical data record using optical character recognition by the processing unit (201).

7. The method according to one of the claims 1 to 3, wherein the data record identifier is encoded in a one-dimensional or two-dimensional code in the first medical record, and wherein the data record identifier is extracted from the one-dimensional or two-dimensional code by the processing unit (201).

8. The method according to one of the preceding claims, wherein the data record identifier comprises patient name, medical data record number, and/or patient health information.

9. The method according to one of the preceding claims, further comprising:
receiving (801) a request to access the second medical data record by a server (101);
authenticating (802) the request to determine whether the request is received from an authorized source by the server (101); and
providing (803) access to the second medical data record by the server (101), if such a request is from an authorized source.

10. A local transmission unit (101) for providing a second medical data record, the unit comprising:
a first interface (105) configured to receive a first medical data record;
a processing unit (201) configured to determine data record identifier from the first medical data record; furthermore configured to identify the second medical data record within a repository based on the data record identifier, wherein the second medical data record comprises the data record identifier in the repository; furthermore configured to provide the second medical data record; the local transmission (101) unit configured to execute a method according to one of the claims 1 to 9.

11. A computer program product comprising a computer program, the computer program being loadable into a storage unit of a local transmission unit (101), including program code sections to make the local transmission unit (101) execute the method of any one of claims 1 to 9 when the computer program is executed in the local transmission unit (101).

12. A computer-readable medium, on which program code sections of a computer program are saved, the program code sections being loadable into and/or executable in an allocation system to make the local transmission unit (101) execute the method of any one of the claims 1 to 9 when the program code sections are executed in the local transmission unit (101).

## Patentansprüche

1. Verfahren zur Bereitstellung eines zweiten medizinischen Datensatzes, wobei das Verfahren umfasst:
Empfangen (302) eines ersten medizinischen Datensatzes durch eine erste Schnittstelle (105), wobei sich der erste medizinische Datensatz auf einen Patienten bezieht;
Bestimmen (303) eines Datensatzbezeichners aus dem ersten medizinischen Datensatz durch eine Verarbeitungseinheit (201), wobei der Datensatzbezeichner ein eindeutiges numerisches, alphabetisches oder alphanumerisches Kennzeichen zur Unterscheidung eines medizinischen Datensatzes von einem anderen ist;
Identifizieren (304) des zweiten medizinischen Datensatzes innerhalb eines Repositoriums auf der Grundlage des Datensatzbezeichners durch die Verarbeitungseinheit (201), wobei der zweite medizinische Datensatz in dem Repositorium den Datensatzbezeichner umfasst; und
Bereitstellung (305) des zweiten medizinischen Datensatzes über die erste Schnittstelle (105),
Ergänzen (306) von Daten aus dem zweiten medizinischen Datensatz mit dem ersten medizinischen Datensatz durch die Verarbeitungseinheit (201),
wobei das Verfahren beim Ergänzen (306) von Daten aus dem zweiten medizinischen Datensatz umfasst:
Identifizieren eines ersten Attributs in einem ersten medizinischen Bild, das mit dem ersten medizinischen Datensatz verbunden ist, wobei das erste Attribut eine Patientenidentifikationsnummer oder die Art der Bildgebungsmodalität sein kann;
Bestimmung eines zweiten medizinischen Bildes im zweiten medizinischen Datensatz, das dem ersten medizinischen Bild im ersten medizinischen Datensatz entspricht, basierend auf dem ersten Attribut;
Extrahieren des zweiten medizinischen Bildes aus dem zweiten medizinischen Datensatz; und
Ergänzen des ersten medizinischen Datensatzes mit dem zweiten medizinischen Bild.

2. Verfahren nach Anspruch 1, wobei der erste medizinische Datensatz ein Bild eines physischen medizinischen Datensatzes umfasst, wobei das Bild ein digitales Bild ist.

3. Verfahren nach Anspruch 1, wobei das Verfahren beim Empfangen des ersten medizinischen Datensatzes das Erfassen (301) eines digitalen Bildes des physischen medizinischen Datensatzes durch ein Client-Gerät (107) umfasst.

4. Verfahren nach Anspruch 2, wobei das Verfahren bei der Bestimmung des Datensatzbezeichners umfasst:
Verarbeiten (401) des ersten medizinischen Datensatzes durch die Verarbeitungseinheit (201);
Identifizieren (402) eines Abschnitts des ersten medizinischen Datensatzes, der den Datensatzbezeichner enthält, aus dem Bild des ersten medizinischen Datensatzes durch die Verarbeitungseinheit (201); und
Extrahieren (403) des Datensatzbezeichners aus dem identifizierten Abschnitt durch die Verarbeitungseinheit (201) .

5. Verfahren nach einem der vorhergehenden Ansprüche, wobei der Datensatzbezeichner in dem Teil des ersten medizinischen Datensatzes durch Extraktion von Textinformationen aus dem Bild des ersten medizinischen Datensatzes identifiziert wird.

6. Verfahren nach Anspruch 5, wobei das Extrahieren von Textinformationen umfasst:
Erkennen (501) des Vorhandenseins eines Textes in dem ersten medizinischen Datensatz durch die Verarbeitungseinheit (201);
Lokalisieren (502) des Textes im ersten medizinischen Datensatz durch die Verarbeitungseinheit (201);
Extrahieren (503) des Textes im ersten medizinischen Datensatz durch die Verarbeitungseinheit (201); und
Erkennen (504) des Textes im ersten medizinischen Datensatz mittels optischer Zeichenerkennung durch die Verarbeitungseinheit (201).

7. Verfahren nach einem der Ansprüche 1 bis 3, wobei der Datensatzbezeichner in einem eindimensionalen oder zweidimensionalen Code in dem ersten medizinischen Datensatz kodiert ist und wobei der Datensatzbezeichner von der Verarbeitungseinheit (201) aus dem eindimensionalen oder zweidimensionalen Code extrahiert wird.

8. Verfahren nach einem der vorhergehenden Ansprüche, wobei der Datensatzbezeichner den Patientennamen, die Nummer des medizinischen Datensatzes und/oder die Gesundheitsinformationen des Patienten umfasst.

9. Verfahren nach einem der vorangehenden Ansprüche, das ferner umfasst:
Empfangen (801) einer Anfrage zum Zugriff auf den zweiten medizinischen Datensatz durch einen Server (101);
Authentifizieren (802) der Anforderung, um festzustellen, ob die Anforderung von einer autorisierten Quelle durch den Server (101) empfangen wird; und
Bereitstellen (803) des Zugriffs auf den zweiten medizinischen Datensatz durch den Server (101), wenn eine solche Anfrage von einer autorisierten Quelle stammt.

10. Eine lokale Übertragungseinheit (101) zum Bereitstellen eines zweiten medizinischen Datensatzes, wobei die Einheit umfasst:
eine erste Schnittstelle (105), die zum Empfang eines ersten medizinischen Datensatzes konfiguriert ist;
eine Verarbeitungseinheit (201), die so konfiguriert ist, dass sie den Datensatzbezeichner aus dem ersten medizinischen Datensatz bestimmt;
ferner konfiguriert, um den zweiten medizinischen Datensatz in einem Repository auf der Grundlage des Datensatzbezeichners zu identifizieren, wobei der zweite medizinische Datensatz den Datensatzbezeichner im Repository umfasst;
ferner konfiguriert, um den zweiten medizinischen Datensatz bereitzustellen; wobei die lokale Übertragungseinheit (101) konfiguriert ist, um ein Verfahren nach einem der Ansprüche 1 bis 9 auszuführen.

11. Computerprogrammprodukt mit einem Computerprogramm, wobei das Computerprogramm in eine Speichereinheit einer lokalen Übertragungseinheit (101) geladen werden kann und Programmcodeabschnitte enthält, um die lokale Übertragungseinheit (101) zu veranlassen, das Verfahren nach einem der Ansprüche 1 bis 9 auszuführen, wenn das Computerprogramm in der lokalen Übertragungseinheit (101) ausgeführt wird.

12. Computerlesbares Medium, auf dem
Programmcodeabschnitte eines Computerprogramms gespeichert sind, wobei die Programmcodeabschnitte in ein Zuordnungssystem ladbar und/oder ausführbar sind, um die lokale Übertragungseinheit (101) zu veranlassen, das Verfahren nach einem der Ansprüche 1 bis 9 auszuführen, wenn die Programmcodeabschnitte in der lokalen Übertragungseinheit (101) ausgeführt werden.

## Revendications

1. Méthode de fourniture d'un deuxième enregistrement de données médicales, comprenant les éléments suivants
réception (302) d'un premier enregistrement de données médicales par une première interface (105), le premier enregistrement de données médicales étant lié à un patient ;
déterminer (303) un identifiant d'enregistrement de données à partir du premier enregistrement de données médicales par une unité de traitement (201), l'identifiant d'enregistrement de données étant une étiquette numérique, alphabétique ou alphanumérique unique permettant de différencier un enregistrement de données médicales d'un autre ;
identifier (304) le second enregistrement de données médicales dans un référentiel sur la base de l'identifiant de l'enregistrement de données par l'unité de traitement (201), le second enregistrement de données médicales dans le référentiel comprenant l'identifiant de l'enregistrement de données ; et
fournir (305) le deuxième enregistrement de données médicales par la première interface (105),
augmenter (306) de données provenant du deuxième enregistrement de données médicales au premier enregistrement de données médicales par l'unité de traitement (201),
dans lequel, en augmentant (306) les données du second enregistrement de données médicales, la méthode comprend :
identifier un premier attribut dans une première image médicale associée au premier enregistrement de données médicales, le premier attribut pouvant être un numéro d'identification du patient ou un type de modalité d'imagerie ;
déterminer une deuxième image médicale dans le deuxième enregistrement de données médicales correspondant à la première image médicale dans le premier enregistrement de données médicales sur la base du premier attribut ;
extraire la seconde image médicale du second enregistrement de données médicales ; et
compléter le premier enregistrement de données médicales par la seconde image médicale.

2. Méthode selon la revendication 1, dans laquelle le premier enregistrement de données médicales comprend une image d'un enregistrement de données médicales physique, l'image étant une image numérique.

3. Méthode selon la revendication 1, dans laquelle, lors de la réception du premier enregistrement de données médicales, la méthode comprend la capture (301) d'une image numérique de l'enregistrement physique de données médicales par un dispositif client (107).

4. Méthode selon la revendication 2, dans laquelle la méthode consiste à déterminer l'identifiant d'enregistrement de données :
traitement (401) du premier enregistrement de données médicales par l'unité de traitement (201) ;
identifier (402) une partie du premier enregistrement de données médicales contenant l'identifiant d'enregistrement de données à partir de l'image du premier enregistrement de données médicales par l'unité de traitement (201) ; et
extraction (403) de l'identifiant d'enregistrement de données de la partie identifiée par l'unité de traitement (201) .

5. Méthode selon l'une des revendications précédentes, dans laquelle l'identifiant d'enregistrement de données est identifié dans la partie du premier enregistrement médical par l'extraction d'informations textuelles de l'image du premier enregistrement de données médicales.

6. Méthode selon la revendication 5, dans laquelle
l'extraction d'informations textuelles comprend :
détection (501) de la présence d'un texte dans le premier enregistrement de données médicales par l'unité de traitement (201) ;
localisation (502) du texte dans le premier enregistrement de données médicales par l'unité de traitement (201) ;
extraction (503) du texte dans le premier enregistrement de données médicales par l'unité de traitement (201) ; et
reconnaître (504) le texte du premier enregistrement de données médicales à l'aide de la reconnaissance optique de caractères par l'unité de traitement (201).

7. Méthode selon l'une des revendications 1 à 3, dans laquelle l'identifiant d'enregistrement de données est codé dans un code unidimensionnel ou bidimensionnel dans le premier enregistrement de données médicales, et dans laquelle l'identifiant d'enregistrement de données est extrait du code unidimensionnel ou bidimensionnel par l'unité de traitement (201).

8. Méthode selon l'une des revendications précédentes, dans laquelle l'identifiant d'enregistrement de données comprend le nom du patient, le numéro de l'enregistrement de données médicales et/ou des informations sur la santé du patient.

9. Méthode selon l'une des revendications précédentes, comprenant en outre :
réception (801) d'une demande d'accès au deuxième dossier médical par un serveur (101) ;
authentifier (802) la demande pour déterminer si elle a été reçue par le serveur (101) en provenance d'une source autorisée ; et
permettre (803) au serveur (101) d'accéder au deuxième enregistrement de données médicales, si la demande émane d'une source autorisée.

10. Unité de transmission locale (101) pour fournir un deuxième enregistrement de données médicales, l'unité comprenant :
une première interface (105) configurée pour recevoir un premier enregistrement de données médicales ;
une unité de traitement (201) configurée pour déterminer l'identifiant de l'enregistrement de données à partir du premier enregistrement de données médicales ; configuré en outre pour identifier le deuxième enregistrement de données médicales dans un référentiel sur la base de l'identifiant d'enregistrement de données, le deuxième enregistrement de données médicales comprenant l'identifiant d'enregistrement de données dans le référentiel ;
configuré en outre pour fournier le deuxième enregistrement de données médicales; l'unité de transmission locale (101) configurée pour exécuter un procédé selon l'une des revendications 1 à 9.

11. Produit programme d'ordinateur comprenant un programme d'ordinateur, le programme d'ordinateur pouvant être chargé dans une unité de stockage d'une unité de transmission locale (101), comprenant des sections de code de programme pour que l'unité de transmission locale (101) exécute la méthode de l'une des revendications 1 à 9 lorsque le programme d'ordinateur est exécuté dans l'unité de transmission locale (101).

12. Support lisible par ordinateur, sur lequel des sections de code de programme d'un programme d'ordinateur sont sauvegardées, les sections de code de programme pouvant être chargées et/ou exécutées dans un système d'attribution pour que l'unité de transmission locale (101) exécute la méthode de l'une quelconque des revendications 1 à 9 lorsque les sections de code de programme sont exécutées dans l'unité de transmission locale (101).
